(19) Europäisches Patentamt
European Patent Office
Office européen des brevets

(11) Veröffentlichungsnummer : **0 097 940**
**B1**

(12) **EUROPÄISCHE PATENTSCHRIFT**

(45) Veröffentlichungstag der Patentschrift : **24.04.85**

(21) Anmeldenummer : **83106222.9**

(22) Anmeldetag : **25.06.83**

(51) Int. Cl.⁴ : **C 07 C 29/136**, C 07 C 29/14, C 07 C 79/22, C 07 D307/42, C 07 D333/16, C 07 D207/32, C 07 D233/54, C 07 D249/08

# (54) Verfahren zur Herstellung araliphatischer Alkohole.

(30) Priorität : **30.06.82 DE 3224380**

(43) Veröffentlichungstag der Anmeldung : **11.01.84 Patentblatt 84/02**

(45) Bekanntmachung des Hinweises auf die Patenterteilung : **24.04.85 Patentblatt 85/17**

(84) Benannte Vertragsstaaten : **BE CH DE FR GB IT LI NL**

(56) Entgegenhaltungen : **FR-A- 1 550 129**
**GB-A- 1 373 303**

(73) Patentinhaber : **HOECHST AKTIENGESELLSCHAFT**
**Postfach 80 03 20**
**D-6230 Frankfurt am Main 80 (DE)**

(72) Erfinder : **Spielmann, Werner, Dr.**
**Johann-Strauss-Strasse 18a**
**D-6233 Kelkheim (Taunus) (DE)**
Erfinder : **Schaeffer, Georg, Dr.**
**Herderstrasse 58**
**D-6238 Hofheim am Taunus (DE)**

**EP 0 097 940 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Unter araliphatischen Alkoholen werden hier Verbindungen der nachstehenden allgemeinen Formel verstanden :

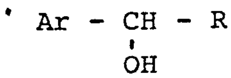

worin Ar = aromatischer oder heterocyclischer Rest, und R = H, ein aliphatischer oder — wie Ar — ebenfalls ein aromatischer oder heterocyclischer Rest.

Sie sind hauptsächlich Zwischenprodukte auf verschiedenen Sachgebieten wie z. B. dem Pharma- und Pflanzenschutzmittel-Sektor.

Ihre Herstellung kann nach einer Reihe verschiedener bekannter Methoden erfolgen. Eine solche Methode — nach der im übrigen Alkohole ganz allgemein herstellbar sind — ist die Reduktion der entsprechenden Aldehyde und Ketone mit einem Aluminiumalkoholat, vorzugsweise in dem dem Alkoholat zugrundeliegenden Alkohol, nach Meerwein-Ponndorf-Verley ; vergl. den zusammenfassenden Artikel von A. L. Wildsin Organic Reactions Vol. II (1944) S. 178-223. Mit dem als Alumininiumalkoholat bevorzugten Aluminiumisopropylat als Reduktionsmittel und Katalysator läßt sich die Reaktion formelmäßig wie folgt darstellen :

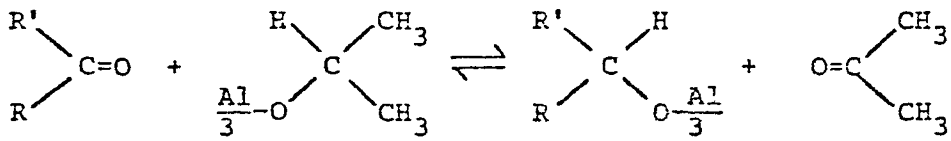

(R ist hier H oder ein organischer Rest,
R' ein organischer Rest).

Nach dem vorerwähnten sowie auch dem übrigen einschlägigen Stand der Technik liefert das Verfahren jedoch hohe bis praktisch quantitative Alkohol-Ausbeuten nur dann, wenn (wegen des Charakters der Reaktion als einer Gleichgewichtsreaktion) die aus dem eingesetzten Aluminiumalkoholat entstehende Carbonylverbindung (Aldehyd oder Keton) während der Reaktion laufend abdestilliert und damit aus dem Gleichgewicht entfernt wird. Geschieht letzteres nicht, so werden wegen der entsprechenden Gleichgewichtseinstellung nur unbefriedigende Alkohol-Ausbeuten erzielt.

Dies zeigt sich etwa bei dem in der FR-A-1 550 129 beschriebenen Beispiele IVb, in welchem 2',5'-Dichlorphenyl-2,2-dichloracetophenon mit Aluminiumisopropylat/Isopropanol 12 Stunden unter Rückfluß — also ohne laufende Abdestillation des aus dem Al-isopropylat entstehenden Acetons — gehalten und erst danach, aber noch vor der hydrolytischen Aufarbeitung des Reaktionsansatzes, abdestilliert wird. Nach der Abdestillation des Acetons wird der auf 20 °C abgekühlte Reaktionsansatz auf Eis gegossen und mit Salzsäure versetzt, dann mit Methylenchlorid extrahiert, der Methylenchloridextrakt getrocknet und destilliert. Das Reaktionsprodukt 1-(2',5'-Dichlorphenyl)-2,2-dichlorethanol wird dabei — wie sich aus den in dem Beispiel mitgeteilten Zahlenangaben ergibt — in einer Ausbeute von nur 52,2 % d. Th. erhalten.

Infolge des Charakters der Meerwein-Pondorf-Verley-Reaktion als einer Gleichgewichtsreaktion ist es auch möglich, sie entgegen der Reduktion von Aldehyden und Ketonen zu den entsprechenden Alkoholen nach Meerwein-Ponndorf-Verley zur Oxidation von Alkoholen zu den entsprechenden Aldehyden und Ketonen zu lenken ; in dieser Form ist die Reaktion als sog. Oppenauer-Oxidation bekannt geworden.

Die Menge des bei der Reduktion nach Meerwein-Ponndorf-Verley eingesetzten Aluminiumalkoholats schwankt nach den Literaturangaben und ist danach weitgehend abhängig von der Art der eingesetzten Ausgangs-Carbonylverbindung. Je geringer die eingesetzte Alkoholatmenge, desto länger ist im allgemeinen die Reaktionszeit, und je höher die Alkoholatmenge, desto kürzer die Reaktionszeit.

Die Verwendung geringer — d. h. unterstöchiometrischer — Alkoholatmengen ist hauptsächlich bei der Reduktion von Aldehyden angezeigt, weil da beim Einsatz höherer Alkoholatmengen die Nebenreaktionen (Kondensationsreaktionen) stark zunehmen. Die vollständige Reduktion beispielsweise des Benzaldehyds unter Einsatz der halben stöchiometrischen Menge Aluminiumisopropylat (etwa 0,17 Mol/Mol Aldehyd) in Isopropanol erfordet nach der vorerwähnten Literatur (Organic Reactions Vol. 2, S. 201/2) eine Reaktionszeit von etwa 6 Stunden.

Für die Reduktion von Ketonen, die unter dem Einfluß alkalischer Reagentien (wie der Aluminiumalkoholate) weniger leicht Kondensationsreaktionen eingehen als die Aldehyde, werden nach der vorerwähnten Literatur (Organic Reactions Vol. 2, S. 195) zur Vermeidung unwirtschaftlich langer Reaktionszeiten mindestens etwa 100 bis 200 %ige Alkoholatüberschüsse empfohlen ; 100 %ige Alkoholatüberschuß bedeutet 2/3 Mol Aluminiumalkoholat pro Mol Ausgangs-Carbonylverbindung. Immerhin ist aber

beispielsweise für die Reduktion des 1-Keto-1,2,3,4-tetrahydrophenanthrens mit einem 100 %igen Alkoholatüberschuß bis zur Reaktionsbeendigung ebenfalls noch eine Reaktionszeit von etwa 6 Stunden erforderlich ; mit einem 800 %igen Alkoholatüberschuß (= 3 Mol Aluminiumalkoholat/Mol Keton) sinkt die Reaktionszeit auf etwa 45 Min. (siehe a.a.O.).

Auf die Ausbeute soll die Reaktionszeit — solange nur die Reduktion immer zu Ende geführt wird — nur einen geringen bzw. praktisch keinen Einfluß haben.

Obwohl die Reduktion nach Meerwein-Ponndorff-Verley ein sehr selektives und — u. a. auch wegen der leichten Zugänglichkeit und des niedrigen Preises der Aluminiumalkoholate — ein wirtschaftliches Verfahren ist, besitzt das Verfahren insbesondere im Hinblick auf seine technische Anwendung jedoch auch Nachteile.

Diese sind hauptsächlich einmal die Notwendigkeit zweier Destillationsschritte (1. der laufenden Abdestillation der aus dem Aluminiumalkoholat stammenden Carbonylverbindung, 2. der Destillation des gewünschten Alkohols im Zuge der Aufarbeitung des Reaktionsansatzes) ; zum anderen wird vor allem bei der Reduktion der Ketone (was höhere Alkoholatüberschüsse erfordert) der Kostenvorteil des billigen Aluminiumalkoholats durch den erheblichen Überschuß wieder kompensiert oder sogar überkompensiert.

Es bestand daher die Aufgabe, die an sich günstige Reduktionsmethode nach Meerwein-Ponndorf-Verley noch weiter zu verbessern.

Diese Aufgabe konnte erfindungsgemäß bei der Herstellung araliphatischer Alkohole der auf Seite 1 angegebenen allgemeinen Formel dadurch gelöst werden, daß man die bei der Reduktion nach Meerwein-Ponndorf-Verley aus den eingesetzten Aluminiumalkoholaten entstehenden Aldehyde oder Ketone erst nach der Hydrolyse der Reaktionsmischung im Zuge der Destillation der organischen Phase entfernt. Vorzugsweise wird hierbei eine nicht höhere als etwa stöchiometrische Menge Aluminiumalkoholat, d. h. nicht mehr als etwa 0,33 Mol pro Mol Ausgangs-Aldehyd oder -Keton, insbesondere nur eine katalytische Alkoholatmenge, eingesetzt.

Erfindungsgegenstand ist daher ein Verfahren zur Herstellung araliphatischer Alkohole der allgemeinen Formel

$$\mathrm{Ar} - \underset{\displaystyle \mathrm{OH}}{\underset{|}{\mathrm{CH}}} - \mathrm{R}$$

worin Ar = aromatischer oder heterocyclischer Rest, R = H, ein aliphatischer oder — wie Ar — ebenfalls ein aromatischer oder heterocyclischer Rest durch Reduktion aromatischer Aldehyde sowie araliphatischer oder aromatischer Ketone mit einem Aluminiumalkoholat, gegebenfalls in Gegenwart eines Alkohols, vorzugsweise des dem Aluminumalkoholat zugrundliegenden Alkohols, nach Meerwein-Ponndorf-Verley, wobei aus dem Aluminiumalkoholat die entsprechenden Aldehyde oder ketone entstehen, und Aufarbeitung auf übliche Weise, vorzugsweise durch Hydrolyse der Reaktionsmischung mit verdünnter Mineralsäure oder verdünnter Alkalilauge, Abtrennung und Destillation der organischen Phase ; das Verfahren ist dadurch gekennzeichnet, daß man die bei der Reduktion aus dem Aluminiumalkoholat entstehenden Aldehyde oder Ketone erst nach der Hydrolyse der Reaktionsmischung im Zuge der Destillation der organischen Phase entfernt.

Dabei können im Prinzip alle möglichen Aluminiumalkoholatmengen eingesetzt werden ; bevorzugt ist jedoch der Einsatz des Aluminiumalkoholats in einer Menge von 0,01 bis 0,33 Mol, vorzugsweise von 0,05 bis 0,1 Mol pro Mol Aldehyd oder Keton, wobei man im Falle der Verwendung der unterstöchiometrischen Alkoholatmengen noch eine entsprechende Menge niederen Alkohols — vorzugsweise des dem Alkoholat zugrundeliegenden Alkoholszusetzt, um die für eine quantitative Umsetezung des Ausgangs-Aldehyds oder -Ketons notwendigen Reduktionsäquivalente zur Verfügung zu stellen.

Bei dieser Verfahrensweise werden praktisch gleiche hohe Ausbeuten — d. h. durchweg nahezu 100 %-erhalten, wie nach der bisherigen Verfahrensweise mit ständiger Abdestillation des aus dem Aluminiumalkoholat entstehenden Aldehyds bzw. Ketons. Dies ist außerordentlich überraschend, weil nach der bisherigen Kenntnis der Reduktion nach Meerwein-Ponndorf-Verley davon auszugehen war, daß ohne die ständige Entfernung des aus dem Aluminiumalkoholat entstehenden Aldehyds bzw. Ketons sowie ganz allgemein ohne dessen Entfernung vor der hydrolytischen Aufarbeitung eine Gleichgewichtsverlagerung auf die Seite des gewünschten Alkohols und damit eine hohe und ausreichende Alkoholausbeute nicht zu erzielen ist.

Für den bevorzugten Einsatz höchstens nur etwa stöchiometrischer Alkoholatmengen (in Kombination mit der Belassung des aus dem Alkoholat entstehenden Aldehyds oder Ketons im Reaktionsansatz) ist weiterhin überraschend, daß hierbei — verglichen mit der bekannten Verfahrensweise nach dem Stand der Technik — bereits nach kürzerer Zeit ein quantitativer Umsatz erreicht wird.

Als Ausgangsverbindungen (aromatische Aldehyde, araliphatische und aromatischer Ketone) für die erfindungsgemäße Verfahrensverbesserung kommen im Prinzip alle möglichen Carbonylverbindungen der nachstehenden allgemeinen Formel in Frage :

$$\mathrm{Ar} - \underset{\displaystyle \mathrm{O}}{\underset{\|}{\mathrm{C}}} - \mathrm{R}$$

worin AR = aromatischer oder heterocyclischer Rest, und R = H, ein aliphatischer, aromatischer oder heterocyclischer Rest.

Bevorzugt sind jedoch aromatische Aldehyde sowie araliphatische und aromatische Ketone der Formel I

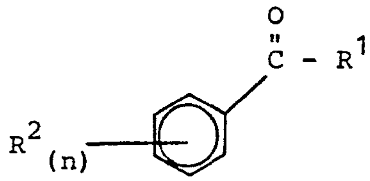

(I)

worin

$R^1$ = H

| | |
|---|---|
| Alkyl, vorzugsw. $C_1$-$C_8$-Alkyl, Alkenyl, vorzugsw. $C_2$-$C_8$-Alkenyl, | ggf. subst. durch Halogen (vorz. F, Cl und/oder Br) oder einen heterocyclischen Rest (vorz. Furyl, Thienyl, Pyrryl, Imidazolyl oder Triazolyl) |
| Aryl, vorzugsw. Phenyl, oder heterocyclischer Rest, vorzugsw. Furyl, Thienyl, Pyrryl, Imidazolyl oder Triazolyl | ggf. subst. durch Halogen (vorz. F, Cl und/oder Br), $NO_2$, Aryl (vorz. Phenyl) oder einen heterocycl. Rest (vorz. Furyl, Thienyl, Pyrryl, Imidazolyl oder Triazolyl) |

$R^2$ = H, Halogen (vorz. F, Cl, Br), $NO_2$, Aryl (vorz. Phenyl) oder heterocyclischer Rest (vorz. Furyl, Thienyl, Pyrryl, Imidazolyl oder Triazolyl), und

n = ganze Zahl von 1-3.

Besonderes bevorzugte Ausgangsstoffe sind die Verbindungen der Formel I mit

$R^1$ = $C_1$-$C_3$-Alkyl (ggf. subst. durch Cl, Br, Imidazolyl oder Triazolyl) oder Phenyl (ggf. subst. durch F, Cl und/oder $NO_2$),

$R^2$ = H, F, Cl und $NO_2$,

n = 1-3

Einige beispielhafte konkrete Ausgangsverbindungen (sowohl nicht bevorzugte als auch bevorzugte und besonderes bevorzugte) für das erfindungsgemäße Verfahren sind :

aromatische Aldehyde :
Benzaldehyd
2-Chlorbenzaldehyd

araliphatische Ketone :
2-Chloracetophenon
2-Chlorpropiophenon
3-Chloracetophenon
2,4-Dichloracetophenon
ω,2,4-Trichloracetophenon
ω-Chlor-4-fluoracetophenon
3-Nitroacetophenon
4-Nitroacetophenon
1-(2,4-Dichlor-benzoylmethyl)-imidazol

aromatische Ketone :
Benzophenon

Als Aluminiumalkoholate sind diejenigen der nachstehenden Formel II bevorzugt

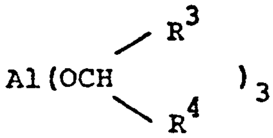

(II)

worin $R^3$ und $R^4$ = unabhängig voneinander H oder $C_1$-$C_4$-Alkyl.

Besonders bevorzugte Aluminiumalkoholate sind Aluminium-isopropylat und -sec. -butylat, vor allem Aluminiumisopropylat.

Die Aluminiumalkoholate können sowohl einzeln als auch in Mischung miteinander eingesetzt werden.

Wenn weniger als die stöchiometrische Alkoholatmenge vergwendet wird, ist der Zusatz noch einer entsprechenden Menge niederen Alkohols (vorzugsweise des dem Alkoholat zugrundeliegenden Alkohols) notwendig, um die für einen quantitativen Umsatz des Ausgangs-Aldehyds oder -Ketons notwendigen Reduktionsäquivalente zur Verfügung zu stellen.

Für die Durchführung der Reaktion ist weiterhin bevorzugt, in einem Lösungsmittel zu arbeiten, wobei als Lösungsmittel (auch wenn überstöchiometrische Aluminiumalkoholatmengen eingesetzt werden) Alkohole- und zwar zweckmäßig der dem jeweiligen Aluminiumalkoholat zugrundeliegende Alkohol -bevorzugt sind. Ebenso können aber auch andere inerte, d. h. die Reaktion nicht störende Lösungsmittel, wie Äther, Kohlenwasserstoffe oder chlorierte Kohlenwasserstoffe (z. B. Toluol, Methylenchlorid, Chloroform) verwendet werden.

Die Reaktionstemperatur liegt im allgemeinen zwischen Raumtemperatur und dem Siedepunkt des verwendeten Lösungsmittels bzw. dem Siedepunkt der Reaktionsmischung. Reaktionstemperaturen über etwa 110 °C sind normalerweise nicht notwendig.

Die Reaktion wird im übrigen wie für die Meerwein-Ponndorf-Verley-Reduktion üblich durchgeführt, nur daß eben die aus dem Aluminiumalkoholat entstehenden Aldehyde oder Ketone während der Reaktion nicht entfernt und vorzugsweise nur niedrige Alkoholatmengen eingesetzt werden. Nach beendeter Reaktion wird wie üblich aufgearbeitet, also zweckmäßig durch Hydrolyse der Reaktionsmischung mit verdünnter Mineralsäure oder verdünnter Alkalilauge, um die entstehenden Aluminiumsalze in Lösung zu halten. Das gewünschte Reaktionsprodukt (araliphatischer Alkohol) wird dann ebenfalls auf übliche Weise, vorzugsweise durch Destillation, gewonnen. Die allgemeine Formel der Produkte ist

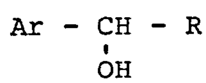

(Ar und R siehe Anfang der Beschreibung) bzw. (wenn man von Carbonylverbindungen der Formel I ausgeht)

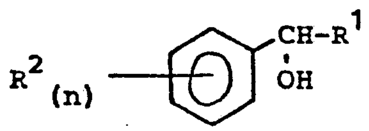

wobei $R^1$, $R^2$ und n die gleiche Bedeutung wie in Formel I besitzen.

Wegen des Wegfalls der Entfernung des Alkoholat-Oxidationsproduktes aus dem Reaktionsgleichgewicht und wegen der z. T. erheblichen Verminderung des Aluminiumalkoholateinsatzes bei gleichzeitiger Verkürzung der Reaktionszeit ohne eine nachteilige Beeinträchtigung des sonstigen Reaktionsablaufs und insbesondere der Produktausbeute stellt die erfindungsgemäße Verbesserung der Meerwein-Ponndorf-Verley-Reaktion einen nicht unerheblichen Fortschritt dar.

Der Erfindung wird nun durch die folgenden Beispiele näher erläutert :

Beispiel 1

In einem trockenen Rührkolben werden unter Ausschluß von Luftfeuchtigkeit zu 74 g handelsüblichem Al-Mischalkoholat (Gemisch aus ca. 42 Mol-% Al-sec-Butylat und ca. 58 Mol-% Al-Isopropylat = 0,33 Mol Al-Alkoholat) bei 50-60 °C eine Schmelze aus 223,5 g (= 1,0 Mol) ω,2,4-Trichloracetophenon (Schmp. 52-55 °C) getropft und 3 Stunden bei 50-60 °C gerührt. Man läßt die Mischung auf 400 ml ca. 2 n Salzsäure fließen, trennt die organische Phase ab und destilliert nach Waschen mit 200 ml 2 %iger $NaHCO_3$-Lösung auf. Man erhält 218,7 g α-Chlormethyl-2,4-dichlorbenzylalkohol (= 97 % d. Th.), Sdp. 144 °C bei 6,6 mbar.

Beispiel 2

Zu einer unter Rückfluß siedenden Lösung aus 40 g handelsüblichem Al-Mischalkoholats (vgl. Beispiel 1) (= 0,18 Mol) in 220 ml Isopropanol tropft man eine Schmelze aus 558,8 g (= 2,5 Mol) ω,2,4-Trichloracetophenon und erhitzt noch 1 Stunde unter Rückfluß bis zum vollständigen Umsatz der Ausgangsverbindung.

Die Reaktionsmischung wird auf 300 ml 7 %ige Salzsäure gegossen und wie in Beispiel 1 aufgearbeitet.

Ausbeute : 218,7 g α-Chlormethyl-2,4-dichlorbenzylalkohol (= 97 % d. Th.)

Beispiel 3

220 ml Isopropanol und 4,9 g Al-Flitter (= 0,18 Mol) werden bis zur Auflösung des Aluminiums unter Rückfluß erhitzt. Anschließend fügt man wie in Beispiel 2 beschrieben 558,8 g ω,2,4-Trichloracetophenon (2,5 Mol) zu und verfährt weiter wie in Beispiel 2.

Ausbeute : 547 g α-Chlormethyl-2,4-dichlorbenzylalkohol (= 97 % d. Th.)

Beispiel 4

223,5 g (= 1,0 Mol) ω,2,4-Trichloracetophenon und 68 g (= 0,33 Mol) Al-Isopropylat werden in 300 ml Toluol 1 Stunde unter Rücklfluß erhitzt. Das Ausgangsprodukt ist dann quantitativ umgesetzt. Man hydrolysiert die Reaktionslösung mit 400 ml 2n Salzsäure und arbeitet wie in Beispiel 1 auf.

Ausbeute : 218,7 g α-Chlormethyl-2,4-dichlorbenzylalkohol (= 97 % d. Th.)

Beispiel 5

223,5 g (= 1,0 Mol) ω,2,4-Trichloracetophenon und 68 g (= 0,33 Mol) Al-Isopropylat werden in 200 ml $CH_2Cl_2$ 4 Std. unter Rückfluß erhitzt. Der Umsatz der Ausgangsverbindung ist dann vollständig. Anschließend arbeitet man wie in Beispiel 4 auf.

Ausbeute : 218,7 g α-Chlormethyl-2,4-dichlorbenzylalkohol (= 97 % d. Th.)

Beispiele 6-14

1 Mol Aldehyd bzw. Keton und 14,4 g Al-Isopropylat (0,07 Mol) wurden in 100 ml Isopropanol unter Rückfluß erhitzt. Nach max. 3 h ist der Aldehyd bzw. das Keton quantitativ zu dem gewünschten Benzylalkohol umgesetzt (GC-Analyse).

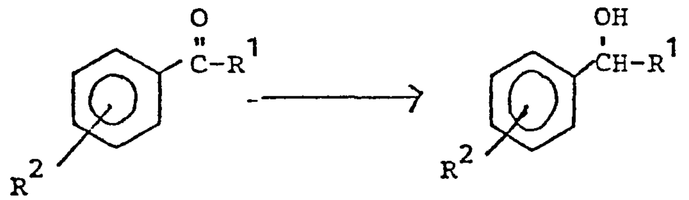

| Beispiel Nr. | $R^1$ | $R^2$ |
|---|---|---|
| 6 | H | H |
| 7 | H | 2-Cl |
| 8 | $CH_3$ | 2-Cl |
| 9 | $C_2H_5$ | 2-Cl |
| 10 | $CH_3$ | 3-Cl |
| 11 | $CH_2Cl$ | 2-Cl. 4-Cl |
| 12 | $CH_2Cl$ | 4-F |
| 13 | $CH_3$ | $3-NO_2$ |
| 14 | $CH_3$ | $4-NO_2$ |

**Patentansprüche**

1. Verfahren zur Herstellung araliphatischer Alkohole der Formel

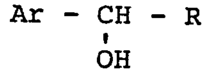

worin Ar = aromatischer oder heterocyclischer Rest, und R = H, ein aliphatischer oder — wie Ar — ebenfalls ein aromatischer oder heterocyclischer Rest, durch Reduktion aromatischer Aldehyde sowie

araliphatischer oder aromatischer Ketone mit einem Aluminiumalkoholat, gegebenenfalls in Gegenwart eines Alkohols, vorzugsweise des dem Aluminiumalkoholat zugrundeliegenden Alkohols, nach Meerwein-Ponndorf-Verley, wobei aus dem Aluminium-alkoholat die entsprechenden Aldehyde oder Ketone entstehen, und Aufarbeitung auf übliche Weise, vorzugsweise durch Hydrolyse der Reaktionsmischung mit verdünnter Mineralsäure oder verdünnter Alkalilauge, Abtrennung und Destillation der organischen Phase, dadurch gekennzeichnet, daß man die bei der Reduktion aus dem Aluminiumalkoholat entstehenden Aldehyde oder Ketone erst nach der Hydrolyse der Reaktionsmischung im Zuge der Destillation der organischen Phase entfernt.

2. Verfahren nach Anspruch 1, dadurch gekennzeichnet, daß man das Aluminiumalkoholat in einer Menge von 0,01 bis 0,33 Mol, vorzugsweise 0,05 bis 0,1 Mol/Mol Aldehyd oder Keton verwendet und daß man im Falle der Verwendung unterstöchiometrischer Alkoholatmengen noch eine entsprechende Menge niederen Alkohols-vorzugsweise des dem Alkoholat zugrundeliegenden Alkohols-zusetzt, um die für eine quantitativen Umsatz des Ausgangs-Aldehyds oder -Ketons notwendigen Reduktions-äquivalente zur Verfügung zu stellen.

3. Verfahren nach den Ansprüchen 1-2, dadurch gekennzeichnet, daß man als aromatische Aldehyde sowie araliphatische und aromatische Ketone Verbindungen der Formel I

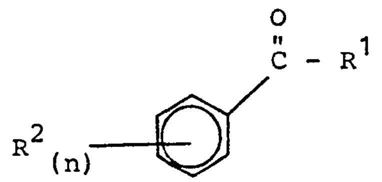

(I)

worin

$R^1$ = H

| | |
|---|---|
| Alkyl, vorz. $C_1$-$C_8$-Alkyl,<br>Alkenyl, vorz. $C_2$-$C_8$-Alkenyl, | ggf. subst. durch Halogen (vorz. F, Cl und/oder Br) oder einen heterocycl. Rest (vorz. Furyl, Thienyl, Pyrryl, Imidazolyl oder Triazolyl) |
| Aryl, vorz. Phenyl, oder heterocycl. Rest, vorz. Furyl, Thienyl, Pyrryl, Imidazolyl oder Triazolyl | ggf. subst. durch Halogen (vorz. F, Cl und/oder Br), $NO_2$, Aryl (vorz. Phenyl) oder einen heterocycl. Rest (vorz. Furyl, Thienyl, Pyrryl, Imidazolyl oder Triazolyl) |

$R^2$ = H, Halogen (vorz. F, Cl, Br), $NO_2$, Aryl (vorz. Phenyl) oder heterocycl. Rest (vorz. Furyl, Thienyl, Pyrryl, Imidazolyl oder Triazolyl), und

n = ganze Zahl von 1-3

verwendet.

4. Verfahren nach den Ansprüchen 1 bis 3, dadurch gekennzeichnet, daß man als Ausgangsverbindungen für die Reduktion Verbindungen der Formel I mit

$R^1$ = $C_1$-$C_3$-Alkyl (ggf. subst. durch Cl, Br, Imidazolyl oder Triazolyl) oder Phenyl (ggf. subst. durch F, Cl und/oder $NO_2$),

$R^2$ = H, F, Cl und $NO_2$,

n = 1-3

verwendet.

5. Verfahren nach den Ansprüchen 1 bis 4, dadurch gekennzeichnet, daß man als Aluminiumalkoholat Aluminiumisopropylat und/oder Aluminium-sec.-Butylat, insbesondere Aluminiumisopropylat, verwendet.

## Claims

1. A process for the preparation of araliphatic alcohols of the formula

$$\mathrm{Ar - \underset{OH}{CH} - R}$$

in which Ar = aromatic or heterocyclic radical, and R = H, an aliphatic or — as Ar — equally an aromatic or heterocyclic radical by reduction of aromatic aldehydes and araliphatic or aromatic ketones with an

aluminum alcoholate, optionally in the presence of an alcohol, preferably the alcohol from which the aluminum alcoholate is derived, by the method of Meerwein-Ponndorf-Verley, the corresponding aldehydes or ketones being produced from the aluminum alcoholate, and working up in usual manner, preferably by hydrolysis of the reaction mixture with aqueous mineral acid or an aqueous alkali base, separation and distillation of the organic phase characterized in removing the aldehydes or ketones being produced from the aluminum alcoholate, during the reduction only after the hydrolysis of the reaction mixture in the course of the distillation of the organic phase.

2. The process as claimed in claim 1, characterized in using the aluminum alcoholate in an mount of 0.01 to 0.33 mole, preferably 0.05 to 0.1 mole per mole of aldehyde or ketone, and in adding in the event of using substoichiometric amounts of the alcoholate still corresponding amount of a lower alcohol, preferably of the alcohol from which the alcoholate is derived in order to have available the reduction equivalents necessary for a quantitative conversion of the starting aldehyde or ketone.

3. The process as claimed in either of claims 1 to 2, characterized in using as the aromatic aldehydes and araliphatic and aromatic ketone compounds of the formula I

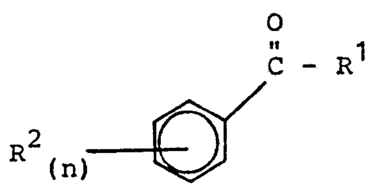

(I)

wherein $R^1$ = H or alkyl, preferably $C_1$-$C_8$-alkyl, alkenyl, preferably $C_2$-$C_8$-alkenyl, all optionally substituted by halogen (preferably F, Cl and/or Br) or a heterocyclic radical (preferably furyl, thienyl, pyrryl, imidazolyl or triazolyl) aryl, preferably phenyl, or a heterocyclic radical, preferably furyl, thienyl, pyrryl, imidazolyl or triazolyl, all optionally substituted by halogen (preferably F, Cl and/or Br), $NO_2$, aryl (preferably phenyl) or a heterocyclic radical (preferably furyl, thienyl, pyrryl, imidazolyl or triazolyl) $R^2$ = H, halogen (preferably F, Cl, Br), $NO_2$, aryl (preferably phenyl) or a heterocyclic radical (preferably furyl, thienyl, pyrryl, imidazolyl or triazolyl) and n = a whole number from 1 to 3.

4. The process as claimed in any of claims 1 to 3, characterized in using as starting compounds for the reduction compounds of the formula I with $R^1$ = $C_1$-$C_3$-alkyl (optionnally substituted by Cl, Br, imidazolyl or triazolyl) or phenyl (optionally substituted by F, Cl and/or $NO_2$), $R^2$ = H, F, Cl and $NO_2$ and n = 1-3.

5. The process as claimed in any of claims 1 to 4, characterized in using as the aluminum alcoholate aluminum isopropylate and/or aluminum sec.-butylate, in particular aluminum isopropylate.

**Revendications**

1. Procédé de préparation d'alcools araliphatiques de formule

$$Ar - \underset{OH}{\underset{|}{CH}} - R$$

dans laquelle Ar représente un radical aromatique ou hétérocyclique, R l'hydrogène, un radical aliphatique ou, de même que Ar, un radical aromatique ou hétérocyclique, par réduction d'aldéhydes aromatiques ou de cétones araliphatiques ou aromatiques avec un alcoolate d'aluminium, éventuellement en présence d'un alcool, de préférence l'alcool correspondant à l'alcoolate, suivant la méthode de Meerwein-Ponndorf-Verley, ce qui forme, à partir de l'alcoolate, les aldéhydes ou cétones correspondant, et traitement du mélange réactionnel de la manière usuelle, de préférence par hydrolyse avec un acide minéral dilué ou une lessive alcaline diluée, puis séparation et distillation de la phase organique, procédé caractérisé en ce que l'on n'élimine qu'après l'hydrolyse du mélange réactionnel, au cours de la distillation de la phase organique, les aldéhydes ou cétones formés dans la réduction à partir de l'alcoolate d'aluminium.

2. Procédé selon la revendication 1, caractérisé en ce que l'on ajoute l'alcoolate d'aluminium dans une proportion de 0,01 à 0,33 mole, de préférence de 0,05 à 0,01 mole, par mole de l'aldéhyde ou de la cétone, et si l'on n'ajoute qu'une proportion de l'alcoolate inférieure à la proportion stœchiométrique, on ajoute encore une quantité correspondante d'un alcool inférieur, de préférence de l'alcool correspondant à l'alcoolate employé, de manière à disposer des équivalents de réduction nécessaires à la conversion quantitative de l'aldéhyde ou de la cétone dont on part.

3. Procédé selon la revendication 1 ou 2, caractérisé en ce que l'on part d'aldéhydes aromatiques ou de cétones araliphatiques ou aromatiques de formule I ci-dessous

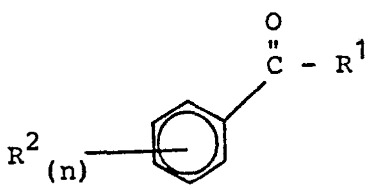

(I)

dans laquelle

$R^1$ désigne l'hydrogène

un alkyle, de préférence en $C_1$-$C_8$, un alcényle, de préférence en $C_2$-$C_8$ — éventuellement substitués par un halogène (de préférence F, Cl et/ou Br) ou un radical hétérocyclique (de préférence le radical furyle, thiényle, pyrryle, imidazolyle ou triazolyle)

un aryle, de préférence un phényle, ou un radical hétérocyclique, de préférence le radical furyle, thiényle, pyrryle, imidazolyle ou triazolyle — éventuellement substitués par un halogène (de préférence F, Cl et/ou Br), $NO_2$, un aryle (de préférence phényle) ou un radical hétérocyclique (de préférence furyle, thiényle, pyrryle, imidazolyle ou triazolyle)

$R^2$ désigne H, un halogène (de préférence F, Cl ou Br), $NO_2$, un aryle (de préférence un phényle) ou un radical hétérocyclique (de préférence furyle, thiényle, pyrryle, imidazolyle ou triazolyle), et

n est un entier de 1 à 3.

4. Procédé selon l'une quelconque des revendications 1 à 3, caractérisé en ce que l'on part de composés de formule I selon la revendication 3 dans lesquels

$R^1$ est un alkyle en $C_1$-$C_3$ (éventuellement substitué par du chlore, du brome ou un groupe imidazolyle ou triazolyle) ou un phényle (éventuellement substitué par du fluor, du chlore et/ou un groupe $NO_2$),

$R^2$ désigne H, F, Cl ou $NO_2$, et

n est un nombre de 1 à 3.

5. Procédé selon l'une quelconque des revendications 1 à 4, caractérisé en ce que l'on utilise comme alcoolate d'aluminium l'isopropylate et/ou le butylate secondaire d'aluminium, mais en particulier l'isopropylate d'aluminium.